# EUROPÄISCHE PATENTSCHRIFT

(11) **EP 3 487 319 B1**
(45) Veröffentlichungstag und Bekanntmachung des Hinweises auf die Patenterteilung: **01.04.2026**
(21) Anmeldenummer: 17740014.0
(22) Anmeldetag: 17.07.2017
(51) Int. Cl.: A61K 8/67, A61K 31/015, A61K 31/05, A61K 31/12, A61K 31/122, A61K 31/235, A61K 31/355, A61K 31/375, A61K 45/06, A61Q 19/00, A61P 3/02, A23P 10/20, A23L 33/15, A23L 33/155, A61K 8/31, A23K 20/174, A61K 8/02

(54) **PROPYLGALLATHALTIGE VITAMINZUBEREITUNGEN**
VITAMIN COMPOSITIONS COMPRISING PROPYL GALLATE
COMPOSITION DE VITAMINE COMPRENANT DE GALLATE DE PROPYLE

(30) Priorität: 19.07.2016 EP 16180199
(43) Veröffentlichungstag der Anmeldung: 29.05.2019
(73) Patentinhaber: BASF SE, 67056 Ludwigshafen am Rhein (DE)
(72) Erfinder: MEYER-BOEHM, Kathrin, 67056 Ludwigshafen (DE); HELGASON, Thrandur, 89257 Illertissen (DE); SINGH, Raajvinder, 67056 Ludwigshafen (DE); DOBLER, Walter, 67056 Ludwigshafen (DE); SCHORDING, Peter, 67308 Bubenheim (DE); WIGBERS, Christof Wilhelm, 67056 Ludwigshafen (DE); KOLTER, Karl, 67056 Ludwigshafen (DE); BRUHNS, Stefan, 67056 Ludwigshafen (DE); PELLETIER, Wolf, 68623 Lampertheim (DE); WAGNER, Daniel, 68623 Lampertheim (DE)
(74) Vertreter: BASF IP Association
(86) Internationale Anmeldenummer: PCT/EP2017/068038
(87) Internationale Veröffentlichungsnummer: WO 2018/015346

(56) Entgegenhaltungen:
- EP-A2- 0 065 193
- CN-A- 102 657 663
- DE-A1- 19 651 681
- DE-A1- 19 903 716
- DE-T2- 69 728 206
- US-A- 2 756 177
- US-A- 2 933 392
- DZIEZAK ET AL: "Preservatives: antioxidants. The ultimate answer to oxidation", FOOD TECHNOL, INSTITUTE OF FOOD TECHNOLOGISTS, CHICAGO, IL, US, vol. 40, no. 9, 1 January 1986 (1986-01-01), pages 94 - 102, XP008135216, ISSN: 0015-6639
- DATABASE WPI Week 201306, Derwent World Patents Index; AN 2012-R30178

## Beschreibung

Die vorliegende Erfindung betrifft eine Zubereitung mit antioxidativen Eigenschaften, deren Herstellung und Verwendung.

Eine Schwierigkeit bei der Herstellung vitaminhaltiger Zubereitungen ist, dass diese sowohl während des Herstellungsprozesses als auch bei der folgenden Lagerung oder Weiterverarbeitung in Prämixen, Pellets oder Futtern oftmals nicht stabil sind und durch oxidative Prozesse geschädigt werden. Die Schädigungen können durch Reaktion mit Luftsauerstoff oder Interaktionen mit Schwermetallen oder durch die Absorption von UV-Strahlung verursacht werden. Durch die hierdurch bedingten Schädigungen können die Vitamine z.B. ihre Farbe ändern und/oder ihre Wirksamkeit verlieren.

Ein bekannter Weg, die beschriebenen Probleme zu behandeln, besteht in einem Zusatz von Antioxidantien zu den Zubereitungen.

Laut CD Römpp Chemie Lexikon 10. Auflage Version 1.3, Stuttgart/New York: Georg Thieme Verlag handelt es sich bei Antioxidantien um Verbindungen, die unerwünschte, durch Sauerstoff-Einwirkungen u.a. oxidative Prozesse bedingte Veränderungen in den zu schützenden Stoffen hemmen oder verhindern.

Antioxidativ wirksam und damit schützend wirksam bei Vitaminen ist Ethoxyquin. Die toxikologische Datenlage zu Ethoxyquin ist unzureichend, die Substanz Ethoxyquin selbst gilt als nicht genotoxisch. Die European Food Safety Authority (EFSA) hat jedoch festgestellt, dass einer seiner Metaboliten, Ethoxyquin-Chinonimin, genotoxisch (d.h. DNA schädigend) sein könnte, was auf mögliche Sicherheitsbedenken schließen lässt.

Infolge des Herstellungsprozesses von Ethoxyquin verbleibt in dem Futterzusatzstoff zudem eine Verunreinigung, p-Phenetidin, bei der es sich möglicherweise um ein Mutagen handelt. Mutagene sind Stoffe, die Mutationen im Erbgut von Tieren und Menschen auslösen.

Die EFSA kann aufgrund der momentan nicht vorhandenen Datenlage nicht über die Sicherheit von Ethoxyquin als Futtermittelzusatz für die Zieltiere befinden und auch keine abschließende Bewertung der Sicherheit für Verbraucher oder die Umwelt vornehmen.

Aufgrund dieser Sicherheitsbedenken besteht akuter Bedarf nach Antioxidantien, die sich für den Einsatz als Zusatzstoff für Tierfutter, Lebensmittel, Nahrungsergänzungsmitteln, Körperpflegeprodukten oder pharmazeutischen Mittel eignen und Ethoxyquin kurzfristig in seinen Zubereitungen ersetzen können.

EP 0065193 offenbart ein Verfahren zur Herstellung von feinverteilten, pulverförmigen Carotinoid bzw. Retinoidpräparaten. Hieraus ist bekannt, dass Antioxidantien bei der Herstellung von Wirkstoffzubereitungen entweder der wässrigen oder der wirkstoffhaltigen Lösungsmittelphase zugesetzt werden können, sie jedoch vorzugweise gemeinsam mit dem Wirkstoff und optional tensidartigen Stabilisatoren in der Lösungsmittelphase vorzumischen sind.

EP 0807431(eq. DE 69728206) offenbart ein Verfahren zur Herstellung eines Pulvers, wobei die mittlere Partikelgröße der ins Pulver eingebetteten Substanz 0.1 bis 0.5µm beträgt. Bevorzugt handelt es sich bei der eingebetteten Substanz um ein Carotenoid.

Aufgabe der Erfindung ist es daher, eine Zubereitung zur Verfügung zu stellen, die die Stabilitäten von Ethoxyquin enthaltenden Formulierungen erreicht bzw. übertrifft, ohne die für Ethoxyquin existierenden Sicherheitsbedenken aufzuwerfen.

Hierbei wurde überraschend gefunden, dass eine pulverförmige Vitaminzubereitung, in der das Vitamin im Wesentlichen eine Teilchengrösse von weniger als 0,7 µm besitzt und die eine wirksame Menge Propylgallat enthält, hervorragend geeignet ist, die gestellte Aufgabe zu lösen. Dabei beträgt die wirksame Menge Propylgallat 3,5 bis 9,5 Gew.-% bezogen auf die Gesamtmenge der Zubereitung, wobei das Gewichtsverhältnis von Propylgallat zu Vitamin bei der Herstellung zwischen 0,21 und 2,63 liegt.

Unter wirksamer Menge Propylgallat ist dabei erfindungsgemäß dieMenge an Propylgallat zu verstehen, die geeignet ist das Vitamin in der Zubereitung so zu stabilisieren, dass nach 4 Wochen im Stresstest noch mindestens 20 Gew-% Vitamin, bevorzugt mindestens 25 Gew-% und insbesondere bevorzugt mindestens 30 Gew-% des zu Beginn des Stresstests bestimmten Vitamingehalts erhalten sind.

Der Stresstest für die Vitamine ist so ausgestaltet, dass Muster von jeweils 100 mg der hergestellten Zubereitung und 4 g Prämixmischung in Glasbehälter eingewogen werden. Die Prämixmischung besteht aus 50 Gew.-% Feinkalk (Partikelgrösse <1000µm), 20 Gew.-% Weizengrießkleie (Partikelgrösse <1000µm), 20 Gew.-% 50%-igem auf Kieselsäure geträgertem Cholinchlorid (Partikelgrösse <1000µm) und 10 Gew-% Spurenelementmischung (Partikelgrösse 100-500µm). Die Spurenelementmischung setzt sich zusammen aus 46,78 Gew-% FeSO₄x7H₂O (100-500µm), 37,43 Gew-% CuSO₄x5H₂O (100-500µm), 11,79 Gew-% ZnO (<500µm), 3,61 Gew-% MnO und 0,39 Gew-% CoCO₃. Nach Zugabe aller Inhaltsstoffe werden die Muster sorgfältig gemischt, wobei die Mischung mechanisch oder auch per Hand durchgeführt wird, und diese Muster (A) in einer Klimakammer bei 40°C und 70% Luftfeuchte für 4 Wochen gelagert. Vor Beginn der Lagerung und nach Abschluss der Lagerung wird der Vitamingehalt der Muster bestimmt. Aus dem Verhältnis der Gehalte nach (A2), und vor der Lagerung (A1) wird die Retention (A2/A1) der Proben, d.h. der noch enthaltene Vitamingehalt in den Mustern berechnet.

Unter einer wirksamen Menge Propylgallat sind 3,5 bis 9,5 Gew.-% Propylgallat, vorzugsweise 4 bis 9 Gew.-%, besonders bevorzugt 7 bis 9 Gew.-% und insbesondere 8 bis 9 Gew-% Propylgallat bezogen auf die Gesamtmenge der Zubereitung im Herstellungsschritt zu verstehen, wobei die Summe der Gewichtsprozentangaben der Komponenten 100 Gew-% ergibt und wobei das Gewichtsverhältnis von Propylgallat zu Vitamin bei der Herstellung zwischen 0,21 und 2,63 liegt.

Der Begriff Herstellungsschritt beinhaltet erfindungsgemäß alle Verfahrensschritte a1 bis c1 bzw. a2 bis c2 bzw. a3 bis c3 bis zum Erhalt des gewünschten Verfahrensprodukts.

Explizit betont sei, dass in dieser Anmeldung alle Mengen- und Konzentrationsangaben und Verhältnisse zueinander, unabhängig davon ob sie die pulverförmige Zubereitung oder die Lösung oder Dispersion betreffen, sich auf die eingesetzten Mengen beim Herstellungsschritt beziehen, da sowohl Propylgallat als auch Tocopherol oder Butylhydroxytoluol (BHT) als Antioxidantien bei der Herstellung aber auch der Lagerung oder Weiterverarbeitung einem stetigen Abbauprozess unterliegen und deren Gehalt an der Zubereitung sich daher sicher nur für den Beginn der Herstellung, bei der Einwaage beziffern lässt.

Unter einer Dispersion sind im Rahmen der vorliegenden Erfindung sowohl Emulsion als auch Suspension gemeint.

Der Term "im Wesentlichen" im Sinne dieser Erfindung bedeutet bzw. umfasst insbesondere, dass größer oder gleich 80 Prozent, noch bevorzugt größer oder gleich 85 Prozent, ferner bevorzugt größer oder gleich 90 Prozent sowie am meisten bevorzugt größer oder gleich 95 Prozent der bei der Lösung der pulverförmigen Vitaminzubereitungen gebildeten Teilchen eine Teilchengröße von weniger als 0,7µm besitzen.

Die Bestimmung der Teilchengröße erfolgt dabei durch einen Malvern Zetasizer Nano ZSP.

Bevorzugte Vitamine sind erfindungsgemäß Vitamine ausgewählt aus der Gruppe aus Vitamine D, E, K oder Vitamin Q oder deren Derivate, beispielsweise Vitamin E- Ester wie Tocopherolacetat, Tocotrienol, Vitamin K1, Vitamin K2, Coenzym Q10 sowie Carotinoide, wie β-Carotin, Canthaxanthin, Citranaxanthin, Astaxanthin und Esterderivate, Zeaxanthin und Esterderivate, Lutein und Esterderivate, Lycopin, Apocarotinsäure und Esterderivate, Apocarotinal und Mischungen davon.

Als kohärente Phase enthält die erfindungsgemäße Vitaminzubereitung zumindest ein Kolloid, welches ausgewählt ist aus der Gruppe bestehend aus Pflanzengummis, modifizierten Pflanzengummis, Gelatine, modifizierter Gelatine, modifizierter Stärke, Ligninsulfonat, Chitosan, Carrageenan, Casein, Caseinat, Molkeprotein, Zein, modifizierter Cellulose, Pectin, modifiziertem Pectin, Pflanzenproteinen und modifizierten Pflanzenproteinen oder Mischungen davon.

Unter Pflanzengummis sind dabei erfindungsgemäß zu verstehen Agar, Alginsäure, Alginat, Chicle, Dammar, Eibisch Extrakte, Gellan, Guarkernmehl, Gummi Arabicum, Gummi aus Wegerichkernspelze, Gummi aus Fichtensaft, Johannisbrotkernmehl, Karaya, Konjakmehl, Mastix, Tarakernmehl, Traganth, Xanthan.

Bevorzugt als kohärente Phase sind erfindungsgemäß Gelatine und/oder Pflanzengummis und/oder modifizierte Pflanzengummis.

Überraschend wurde ebenfalls gefunden, dass ein Zusatz von Tocopherol oder BHT zu der Zubereitung in synergistischer Wirkungsweise die antioxidative Schutzwirkung von Propylgallat in der Zubereitung unterstützt oder steigert. Gemäß Erfindung sind unter Tocopherol sowohl das natürliche als auch das synthetische Tocopherol zu verstehen. Unter natürlichem Tocopherol sind die in der Natur vorkommenden α-, β-, γ-, λ-Tocopherole, die auch unter dem Begriff mixed Tocopherole zusammengefasst sind und von der BASF unter dem Markennamen Covi-OX vertrieben werden, zu verstehen. Synthetisches Tocopherol, auch DL-α-Tocopherol genannt, enthält dagegen eine statistische Mischung aus den acht α-Diastereomeren. Bei Zugabe von Tocopherol zu der Zubereitung ist erfindungsgemäß die Zugabe von natürlichem Tocopherol bevorzugt.

Zubereitungen, in denen Propylgallat und Tocopherol in einem Gewichtsverhältnis von 9:1 zu 1:2 zueinander vorliegen sind erfindungsgemäß bevorzugt, wobei ein Verhältnis der beiden Antioxidantien zueinander von 2:1 zu 1:1 besonders bevorzugt ist.

Zubereitungen, in denen durch Zusatz von Butylhydroxytoluol (BHT) die antioxidative Schutzwirkung von Propylgallat unterstützt bzw. gesteigert wird, sind erfindungsgemäß bevorzugt, wenn sie in einem Gewichtsverhältnis Propylgallat zu BHT von 8:1 zu 1:4, besonders bevorzugt, wenn sie in einem Gewichtsverhältnis von 2:1 zu 1:3 zueinander vorliegen.

Der Vorteil der erfindungsgemäßen Zubereitung liegt insbesondere in ihrer antioxidativen Wirkung, der sich im Vergleich mit den Ethoxyquin oder andere übliche Antioxidantien enthaltenden Zubereitungen in einer vergleichbaren, insbesondere verbesserten Stabilität der Vitamine in den erfindungsgemäßen Zubereitungen zeigt.

Die vergleichbare bzw. verbesserte Stabilität der erfindungsgemäßen Vitaminzubereitungen kann anhand eines Stress-Tests demonstriert werden. Dafür werden Muster von jeweils 100 mg der hergestellten Zubereitung und 4 g Prämixmischung in Glasbehälter (50 ml Glasfläschchen) eingewogen. Die Prämixmischung besteht aus 50 Gew.-% Feinkalk (Partikelgrösse <1000µm), 20 Gew.-% Weizengrießkleie (Partikelgrösse <1000µm), 20 Gew.-% 50%-igem auf Kieselsäure geträgertem Cholinchlorid (Partikelgrösse <1000µm) und 10 Gew-% Spurenelementmischung (Partikelgrösse 100-500µm), wobei die Spurenelementmischung sich aus 46,78 Gew-% FeSO₄x7H₂O (100-500µm), 37,43 Gew-% CuSO₄x5H₂O (100-500µm), 11,79 Gew-% ZnO (<500µm), 3,61 Gew-% MnO und 0,39 Gew-% CoCO₃ zusammensetzt. Nach Zugabe aller Inhaltsstoffe werden die Muster sorgfältig gemischt, wobei die Mischung mechanisch oder auch per Hand durchgeführt werden kann, und diese Muster (A) in einer Klimakammer bei 40°C und 70% Luftfeuchte für 4 Wochen gelagert. Ein identischer Test wird mit einer Vergleichsprobe (B) gleicher Zusammensetzung, die aber anstatt Propylgallat die gleiche Menge Ethoxyquin enthält, durchgeführt. Vor Beginn der Lagerung und nach Abschluss der Lagerung wird dabei der Vitamingehalt der Muster bestimmt. Aus dem Verhältnis der Gehalte nach (A2), (B2) und vor der Lagerung (A1) und (B1) wird dabei die Retention (A2/A1) bzw. (B2/B1) der Proben berechnet. Für die erfindungsgemäßen Zubereitungen wird dabei ein Quotient (A2/A1):(B2/B1) von mindestens 0,75 ermittelt. Bevorzugt wird für die erfindungsgemäßen Zubereitungen ein Quotient von mindestens 1 erhalten.

Zur Verbesserung der Herstellung und der Anwendungseigenschaften der Vitaminzubereitung ist es gegebenenfalls zweckmäßig, weitere Komponenten, wie Weichmacher und weitere Hilfs- und Zusatzstoffe zuzusetzen. Bevorzugte Hilfs- und Zusatzstoffe sind dabei Emulgatoren, Öle, wasserlösliche Salze und/oder Trennmittel.

Zur Anpassung der mechanischen Stabilität der kohärenten Phase der Vitaminzubereitung ist es zweckmäßig dem Kolloid zumindest einen Weichmacher zuzusetzen, wie Polyole, Zucker oder Zuckeralkohole, z.B. Saccharose, Glukose, Glukosesirup, Stärkehydrolysate, Fruktose, Fruktosesirup, Lactose, Maltose, Xylose, Arabinose, Ribose, Trehalose, Invertzucker, Sorbit, Mannit, Mannitol, Dextrin, Maltodextrin, Glycerin, Polyetherglykole oder Isomalt. Die Bezeichnung Isomalt steht für einen Zuckeraustauschstoff, der auch unter dem Markennamen Palatinit^{®} (Fa. Südzucker, Deutschland) geführt wird. Isomalt ist eine hydrierte Isomaltulose, die aus etwa gleichen Teilen 6-O-α-D-Glucopyranosyl-D-sorbit und 1-O-α-D-Glucopyranosyl-D-mannit besteht. Bevorzugt verwendete Weichmacher sind Saccharose, Glucosesirup und Lactose.

Weiterhin können als Hilfs- und Zusatzstoffe Emulgatoren zur Stabilisierung der Phasen bei der Herstellung der erfindungsgemäßen Vitaminzubereitung eingesetzt werden, beispielsweise Mono- und Diglyceride, Monoglycerin-Fettsäureester, Polyglycerin-Fettsäureester, Sorbitan-Fettsäureester, Propylenglycol-Fettsäureester, Monoglycerin-Zitronensäureester, Zucker-Fettsäureester oder Lecithin. Bevorzugt verwendete Emulgatoren sind Mono- und Diglyceride, Monoglycerin-Fettsäureester und Lecithin.

Unter Umständen kann es auch vorteilhaft sein, zusätzlich ein physiologisch zugelassenes Öl tierischen oder pflanzlichen Ursprungs zu verwenden, wie beispielsweise Sesamöl, Maiskeimöl, Baumwollsaatöl, Sojabohnenöl, Erdnußöl, Sonnenblumenöl, Rapsöl, Kokosöl, Palmöl, Olivenöl, Distelöl, tierische Fette, Schmalz, Talg, durch Hydrieren, Fraktionieren oder Umesterung modifizierte Öle oder Mischungen. Bevorzugte Öle sind Maiskeimöl, Sonnenblumenöl und Rapsöl. Zusätzlich können der erfindungsgemäßen Vitaminzubereitung noch Metallchelatoren, wie EDTA und Zitronensäure zugesetzt werden.

Weiterhin können für die kohärente Phase der Vitaminzubereitung vorteilhafterweise noch wasserlösliche anorganische und/oder organische Salze zugefügt werden, wie beispielsweise Natriumascorbat, Kaliumascorbat, Calciumascorbat, Natriumerythorbat, Kaliumerythorbat, Natriumbenzoat, Kaliumbenzoat, Natriumcitrat, Kaliumcitrat, Alkaliphosphate, Alkaliacetate, Alkaliphytate und Mischungen davon. Bevorzugte Salze sind Natriumbenzoat und Dinatriumhydrogenphosphat.

Um unerwünschtes Verklumpen zu vermeiden und die Fließfähigkeit zu verbessern, werden zweckmäßigerweise schwer wasserlösliche, feinpartikuläre Trennmittel mit mittleren Partikelgrößen kleiner oder gleich 10µm (x50,3 nach DIN ISO 9276-2:2006-02) zugegeben, die an der Oberfläche der pulverförmigen Vitaminzubereitung anlagern. Das bevorzugte Trennmittel ist dabei ausgewählt aus der Gruppe bestehend aus Siliziumdioxid, hydrophob modifizierte Kiesel säure, Tricalciumphosphat (TCP), Calciumkarbonat, Natriumkarbonat, Kaliumkarbonat, Magnesiumkarbonat, Calciumoxid, Magnesiumoxid, Dicalciumdiphosphat, Calciumsilikat, Magnesiumsilikat, Magnesiumtrisilikat, Natrium-Aluminiumsilikat, Talkum, Kaolin, Calciumstearat, Magnesiumstearat, Stärken der verschiedenen botanischen Quellen, Cellulose oder Mischungen davon. Besonders bevorzugt sind dabei Siliziumdioxid, Tricalciumphosphat (TCP), hydrophob modifizierte Kieselsäure und Maisstärke.

Der Anteil an Hilfs- und Zusatzstoffen ist erfindungsgemäß 0,1 bis 60 Gew.-%, bevorzugt 1 bis 50 Gew.-% und besonders bevorzugt 5 bis 30 Gew.-% bezogen auf die Gesamtmenge der Zubereitung im Herstellungsschritt, wobei die Summe der Gewichtsprozentangaben aller Komponenten der Zubereitung 100 Gew-% ergibt.

Ein weiterer Gegenstand der Erfindung ist ein Verfahren zur Herstellung der oben beschriebenen pulverförmigen Zubereitung umfassend
a1) das Lösen des Vitamins in einem flüchtigen mit Wasser mischbaren organischen Lösungsmittel oder in einer Mischung aus Wasser und einem mit Wasser mischbaren organischen Lösungsmittel bei Temperaturen zwischen 50°C und 200°C, gegebenenfalls bei erhöhtem Druck zwischen 20 und 100bar, innerhalb einer Zeit von weniger als 10 Sekunden
b1) das schnelle Mischen der nach a1) erhaltenen Lösung mit einer wässrigen oder kolloiddispersen Lösung eines Kolloids bei Temperaturen zwischen 0°C und 80°C, wobei das Vitamin in kolloiddisperser Form ausgefällt wird,
c1) Überführung der gebildeten Dispersion in ein Trockenpulver durch Abtrennung der Hauptmenge an Lösungsmittel und anschließender Trocknung.

Das Verfahren wird in Anwesenheit von Propylgallat als Antioxidans durchgeführt, das erfindungsgemäß in einer wirksamen Menge zugesetzt wird. Erfindungsgemäß werden 3,5 bis 9,5 Gew-% Propylgallat bezogen auf die Gesamtmenge der Zubereitung dem Herstellungsverfahren zugesetzt, wobei das Gewichtsverhältnis von Propylgallat zu Vitamin bei der Herstellung zwischen 0,21 und 2,63 liegt. Das Propylgallat kann dabei den Verfahrensschritten a1) und/oder b1) und/oder c1) zugefügt werden, wobei die Zugabe des Propylgallats bei den Verfahrensschritten a1 und/oder b1 bevorzugt ist und die Zugabe zu Verfahrensschritt b1 am bevorzugtesten ist. Bevorzugt wird in dem System Wasser/organisches Lösungsmittel auf einen pH-Wert von 4,5 bis maximal pH 8.5 eingestellt, da bei höheren pH-Werten ein starker Abbau des Propylgallats festzustellen ist. Insbesondere bevorzugt wird ein pH-Wert von 6,5 bis maximal pH 8.5 für das System gewählt.

Bevorzugte Ausführungsformen hinsichtlich der Vitamine finden sich bereits in den eingangs gemachten Erläuterungen.

Die in der Stufe a1) des erfindungsgemäßen Verfahrens verwendeten organischen Lösungsmittel sind vor allem wassermischbare, thermisch stabile, flüchtige, nur Kohlenstoff, Wasserstoff und Sauerstoff enthaltene Lösungsmittel wie Alkohole, Ether, Ester, Ketone oder Acetale. Zweckmäßig verwendet man solche Lösungsmittel, die mindestens zu 10% wassermischbar

sind, einen Siedepunkt unter 200°C aufweisen und/oder weniger als 10 Kohlenstoffatome haben. Besonders bevorzugt werden Methanol, Ethanol, n-Propanol, Isopropanol, 1,2-Butandiol-1-methylether (1-Methoxy-butanol-2), 1,2-Propandiol-1-n-propylether, (1-Propoxy-propanol-2), Tetrahydrofuran, Aceton oder Mischungen davon verwendet.

Als Kolloide werden in dem Verfahren Pflanzengummis, modifizierte Pflanzengummis, Gelatine, modifizierte Gelatine, Stärke, modifizierte Stärke, Ligninsulfonat, Chitosan, Carrageenan, Casein, Caseinat, Molkeprotein, Zein, modifizierte Cellulose, Pectin, modifiziertes Pectin, Pflanzenproteine und modifizierte Pflanzenproteine oder Mischungen davon eingesetzt.

Unter Pflanzengummis sind dabei erfindungsgemäß zu verstehen Agar, Alginsäure, Alginat, Chicle, Dammar, Eibisch Extrakte, Gellan, Guarkernmehl, Gummi Arabicum, Gummi aus Wegerichkernspelze, Gummi aus Fichtensaft, Johannisbrotkernmehl, Karaya, Konjakmehl, Mastix, Tarakernmehl, Traganth, Xanthan.

Bevorzugt sind erfindungsgemäß Gelatine und/oder Pflanzengummis und/oder modifizierte Pflanzengummis.

Zur Erhöhung der mechanischen Stabilität des Trockenprodukts ist es zweckmäßig dem Kolloid einen Weichmacher zuzusetzen, wie Polyole, Zucker oder Zuckeralkohole, z.B. Saccharose, Glukose, Glukosesirup, Stärkehydrolysate, Fruktose, Fruktosesirup, Lactose, Maltose, Xylose, Arabinose, Ribose, Trehalose, Invertzucker, Sorbit, Mannit, Mannitol, Dextrin, Maltodextrin, Glycerin, Polyetherglykole oder Isomalt. Die Bezeichnung Isomalt steht für einen Zuckeraustauschstoff, der auch unter dem Markennamen Palatinit^{®} (Fa. Südzucker, Deutschland) geführt wird. Isomalt ist eine hydrierte Isomaltulose, die aus etwa gleichen Teilen 6-O-α-D-Glucopyranosyl-D-sorbit und 1-O-α-D-Glucopyranosyl-D-mannit besteht. Bevorzugt verwendete Weichmacher sind Saccharose, Glucosesirup und Lactose.

Die erfindungsgemäße pulverförmige Vitaminzubereitung enthält gegebenenfalls auch Emulgatoren, die in ausgesuchten Fällen bevorzugt bei der Herstellung der Dispersion zur Stabilisierung der Phasen bei der Herstellung der erfindungsgemäßen Wirkstoffzubereitung eingesetzt werden. Beispiele sind Mono- und Diglyceride, Monoglycerin-Fettsäureester, Polyglycerin-Fettsäureester, Sorbitan-Fettsäureester, Propylenglycol-Fettsäureester, Monoglycerin-Zitronensäureester, Zucker-Fettsäureester oder Lecithin. Bevorzugt verwendete Emulgatoren sind Mono- und Diglyceride, Monoglycerin-Fettsäureester und Lecithin.

Unter Umständen kann es auch vorteilhaft sein, zusätzlich ein physiologisch zugelassenes Öl tierischen oder pflanzlichen Ursprungs zu verwenden, wie beispielsweise Sesamöl, Maiskeimöl, Baumwollsaatöl, Sojabohnenöl, Erdnußöl, Sonnenblumenöl, Rapsöl, Kokosöl, Palmöl, Olivenöl, Distelöl, tierische Fette, Schmalz, Talg, durch Hydrieren, Fraktionieren oder Umesterung modifizierte Öle oder Mischungen davon. Bevorzugte Öle sind Maiskeimöl, Sonnenblumenöl und Rapsöl.

Zusätzlich kann es vorteilhaft sein im Verfahren Metallchelatoren, wie EDTA und Zitronensäure zuzusetzen.

Das Verhältnis Kolloid und Weichmacher zu Carotinoidlösung wird im Allgemeinen so gewählt, dass ein Trockenprodukt erhalten wird, das zwischen 2 und 25 Gew.-% eines Vitamins, 10 bis 50 Gew.-% eines Kolloids, 20 bis 70 Gew.-% eines Weichmachers und 3,5 bis 9,5 Gew-% Propylgallat, gegebenenfalls entsprechende Mengen Tocopherol oder BHT, sowie 0,1 bis 60-Gew.-% Hilfs- und Zusatzstoffe enthält, wobei die Summe der Prozentangaben aller Komponenten der Zubereitung 100 Gew.-% ergibt.

Das Tocopherol wird bevorzugt dem Verfahrensschritt a1) dem organischen Lösungsmittel zugesetzt, wobei erfindungsgemäß das Verhältnis Propylgallat Tocopherol 9:1 bis 1:2, bevorzugt 2:1 bis 1:1 beträgt. Bei Zugabe von Tocopherol wird erfindungsgemäß bevorzugt natürliches Tocopherol eingesetzt.

Wird BHT zugegeben, erfolgt die Zugabe erfindungsgemäß bevorzugt zur Dispersion, also in Verfahrensschritt c1, nach Abtrennung der Lösungsmittel. Erfindungsgemäß beträgt das Verhältnis Propylgallat BHT 8:1 bis 1:4, bevorzugt 2:1 bis 1:3.

Erfindungsgemäß wird in Schritt c1) des Verfahrens die gebildete Dispersion durch Abtrennung des Lösungsmittels bzw. Gemisches und anschließender Trocknung in ein Trockenpulver überführt. Vorteilhaft ist es, vor der Abtrennung des Lösungsmittels oder Lösungsmittelgemisches den pH-Wert der gebildeten Dispersion mit Schwefelsäure auf einen Bereich von 6,5 bis 7, insbesondere auf pH 6.8 abzusenken, anschließend kann dann die Überführung in ein Trockenpulver u.a. durch Sprühtrocknung, Sprühkühlung, modifizierte Sprühtrocknung, Gefriertrocknung oder Trocknung im Wirbelbett, gegebenenfalls auch in Gegenwart eines Trennmittels erfolgen. Das bevorzugte Trennmittel ist dabei ausgewählt aus der Gruppe bestehend aus Siliziumdioxid, hydrophob modifizierte Kieselsäure, Tricalciumphosphat (TCP), Calciumkarbonat, Natriumkarbonat, Kaliumkarbonat, Magnesiumkarbonat, Calciumoxid, Magnesiumoxid, Dicalciumdiphosphat, Calciumsilikat, Magnesiumsilikat, Magnesiumtrisilikat, Natrium-Aluminiumsilikat, Talkum, Kaolin, Calciumstearat, Magnesiumstearat, Stärken der verschiedenen botanischen Quellen, Cellulose oder Mischungen davon. Besonders bevorzugt sind dabei Siliziumdioxid, Tricalciumphosphat (TCP), hydrophob modifizierte Kieselsäure und Maisstärke.

Bevorzugt wird in Verfahrensschritt c1) die gebildete Dispersion durch destillative Abtrennung der Hauptmenge des Lösungsmittels bzw. Gemisches auf eine Feststoffkonzentration von etwa 25 bis 50 Gew.-% aufkonzentriert und anschließend diese aufkonzentrierte Dispersion in einem Sprühtrockner in ein Trockenpulver überführt.

Besonders bevorzugt wird in Verfahrensschritt c1) des erfindungsgemäßen Verfahrens die Trocknung in einem Sprühtrockner mit integriertem und/oder nachgeschaltetem, externen Wirbelbett durchgeführt. Dabei wird bevorzugt eine pulverförmige Zubereitung mit agglomerierten Partikeln gebildet.

Ein alternatives Verfahren die erfindungsgemäße Zubereitung herzustellen ist, das Vitamin in einem Verfahrensschritt a2) in einem flüchtigen, nicht mit Wasser mischbaren organischen Lösungsmittel bei Temperaturen von 30 bis 150°C, gegebenenfalls unter erhöhtem Druck zu lösen und diese Lösung anschließend in einem Verfahrensschritt b2) in einer wässrigen Lösung eines Kolloids zu emulgieren. Aus der entstandenen Emulsion wird dann in Verfahrensschritt c2) das flüchtige organische Lösungsmittel in an sich bekannter Weise entfernt, z.B. durch Destillation, gegebenenfalls unter Anwendung von vermindertem Druck, wobei eine Dispersion erhalten wird, die durch Abtrennung des Wassers und anschließender Trocknung in ein Trockenpulver überführt werden kann.

Der Begriff "ein nicht mit Wasser mischbares organisches Lösungsmittel" steht im Sinne der vorliegenden Erfindung für ein organisches Lösungsmittel mit einer Wasserlöslichkeit bei Normaldruck von weniger als 10%. Als mögliche Lösungsmittel kommen dabei u.a. halogenierte aliphatische Kohlenwasserstoffe, wie z.B. Methylenchlorid, Chloroform und Tetrachlorkohlenstoff, Carbonsäureester wie Dimethylcarbonat, Diethylcarbonat, Propylencarbonat, Ethylformiat, Methyl-, Ethyl- oder Isopropylacetat sowie Ether wie Methyl-tert. Butylether und entsprechend Mischungen der genannten Lösungsmittel in Frage.

Sollte das Vitamin aufgrund seines niedrigen Schmelzpunktes bei Raumtemperatur (20°C) unter Normaldruck bereits in flüssiger Form vorliegen oder einen Schmelzpunkt unterhalb von 100°C besitzen oder als Lösung in einem Öl vorliegen, ist als weitere Ausgestaltung des Verfahrens möglich das Vitamin direkt bzw. nach dem Aufschmelzen bzw. in Öl gelöst (Verfahrensschritt a3) ohne Verwendung von organischen Lösungsmitteln in einer wässrigen Lösung eines Kolloids zu emulgieren (Verfahrensschritt b3) und anschließend durch Abtrennung des Wassers und anschließender Trocknung (c3) in ein Trockenpulver zu überführen.

Auch in diesen beiden Alternativverfahren ist die Zugabe von Propylgallat vorteilhaft. Analog zu den für Verfahren a1-c1 gemachten Ausführungen wird es in 3,5 bis 9,5 Gew.-% bezogen auf die Gesamtmenge der Zubereitung zugesetzt, wobei das Gewichtsverhältnis von Propylgallat zu Vitamin zwischen 0,21 und 2,63 liegt. Das Propylgallat kann im zuerst genannten Alternativverfahren der wässrigen Kolloidphase, also im Verfahrensschritt b2, dem Vitamin enthaltenden organischen Lösungsmittel (im Verfahrensschritt a2) und/oder der Dispersion (Verfahrensschritt c2) zugefügt werden, wobei die Zugabe des Propylgallats zur wässrigen Kolloidphase und/oder zum Vitamin enthaltenden organischen Lösungsmittel bevorzugt ist und die Zugabe zu Verfahrensschritt b2 am bevorzugtesten ist.

Liegt das Vitamin als Öl bzw. aufgeschmolzen bzw. in einem Öl gelöst vor wird das Propylgallat dem Vitamin (Verfahrensschritt a3) und/oder der wässrigen Kolloidphase (Verfahrensschritt b3) und/oder der Dispersion (Verfahrensschritt c3) zugefügt. Die Zugabe des Propylgallats zur wässrigen Kolloidphase ist hierbei bevorzugtesten.

Bzgl. pH-Wert Einstellung ist zu beachten, dass für den Fall, dass das Propylgallat dem organischen Lösungsmittel bzw. dem Vitamin in Ölform bzw. aufgeschmolzen bzw. in Öl gelöst zugesetzt wird, diese pH-Wert Einstellung in der wässrigen Kolloidphase vorgenommen wird.

Als Öle zur Lösung der Vitamine kommen die in der Anmeldung genannten physiologisch zugelassenen Öle tierischen oder pflanzlichen Ursprungs in Betracht. Bevorzugte Öle hierfür sind Maiskeimöl, Sonnenblumenöl und Rapsöl.

Als Kolloide in dem Verfahren werden Pflanzengummis, modifizierte Pflanzengummis, Gelatine, modifizierte Gelatine, Stärke, modifizierte Stärke, Ligninsulfonat, Chitosan, Carrageenan, Casein, Caseinat, Molkeprotein, Zein, modifizierte Cellulose, Pectin, modifiziertes Pectin, Pflanzenproteine und modifizierte Pflanzenproteine oder Mischungen davon eingesetzt.

Zur Erhöhung der mechanischen Stabilität der Vitaminzubereitung ist es zweckmäßig dem Kolloid während des Verfahrens einen Weichmacher zuzusetzen. Geeignet hierfür sind Polyole, Zucker oder Zuckeralkohole, z.B. Saccharose, Glukose, Glukosesirup, Stärkehydrolysate, Fruktose, Fruktosesirup, Lactose, Maltose, Xylose, Arabinose, Ribose, Trehalose, Invertzucker, Sorbit, Mannit, Mannitol, Dextrin, Maltodextrin, Glycerin, Polyetherglykole oder Isomalt. Die Bezeichnung Isomalt steht für einen Zuckeraustauschstoff, der auch unter dem Markennamen Palatinit^{®} (Fa. Südzucker, Deutschland) geführt wird. Isomalt ist eine hydrierte Isomaltulose, die aus etwa gleichen Teilen 6-O-α-D-Glucopyranosyl-D-sorbit und 1-O-α-D-Glucopyranosyl-D-mannit besteht. Bevorzugt verwendete Weichmacher sind Saccharose, Glucosesirup und Lactose.

Weiterhin können Emulgatoren zur Stabilisierung der Phasen bei der Herstellung der erfindungsgemäßen Wirkstoffzubereitung eingesetzt werden, beispielsweise Mono- und Diglyceride, Monoglycerin-Fettsäureester, Polyglycerin-Fettsäureester, Sorbitan-Fettsäureester, Propylenglycol-Fettsäureester, Monoglycerin-Zitronensäureester, Zucker-Fettsäureester oder Lecithin. Bevorzugt verwendete Emulgatoren sind Mono- und Diglyceride, Monoglycerin-Fettsäureester und Lecithin.

Unter Umständen kann es auch vorteilhaft sein, zusätzlich ein physiologisch zugelassenes Öl tierischen oder pflanzlichen Ursprungs zu verwenden, wie beispielsweise Sesamöl, Maiskeimöl, Baumwollsaatöl, Sojabohnenöl, Erdnußöl, Sonnenblumenöl, Rapsöl, Kokosöl, Palmöl, Olivenöl, Distelöl, tierische Fette, Schmalz, Talg, durch Hydrieren, Fraktionieren oder Umesterung modifizierte Öle oder Mischungen davon. Bevorzugte Öle sind Maiskeimöl, Sonnenblumenöl und Rapsöl.

Zusätzlich kann es vorteilhaft sein im Verfahren Metallchelatoren, wie EDTA und Zitronensäure zuzusetzen.

Das Verhältnis Kolloid und Weichmacher zu Carotinoidlösung wird im Verfahren im allgemeinen so gewählt, dass ein Trockenprodukt erhalten wird, das zwischen 2 und 25 Gew-% eines Vitamins, 10 bis 50 Gew.-% eines Kolloids, 20 bis 70 Gew.-% eines Weichmachers und 3,5 bis 9,5 Gew.-% Propylgallat , gegebenenfalls entsprechende Mengen Tocopherol oder BHT, sowie 0,1 bis 60-Gew.-% Hilfs- und Zusatzstoffe enthält, wobei die Summe der Prozentangaben aller Komponenten der Zubereitung 100 Gew.-% ergibt.

Das Tocopherol wird bevorzugt dem Verfahrensschritt a) dem organischen Lösungsmittel zugesetzt, wobei erfindungsgemäß das Verhältnis Propylgallat Tocopherol 9:1 bis 1:2, bevorzugt 2:1 bis 1:1 beträgt. Bei Zugabe von Tocopherol wird erfindungsgemäß bevorzugt natürliches Tocopherol eingesetzt.

Wird BHT zugegeben, erfolgt die Zugabe erfindungsgemäß bevorzugt zur Dispersion, also in Verfahrensschritt c, nach Abtrennung der Lösungsmittel. Erfindungsgemäß beträgt das Verhältnis Propylgallat BHT 8:1 bis 1:4, bevorzugt 2:1 bis 1:3.

In dem erfindungsgemäßen Verfahren wird in Verfahrensschritt c2) die gebildete Emulsion durch Abtrennung des organischen Lösungsmittels bzw. Gemisches in eine Dispersion überführt und anschließend durch Abtrennung des Wassers mit nachfolgendem Trocknungsschritt in ein Trockenpulver überführt. Vorteilhaft ist es, vor der Abtrennung des Wassers den pH-Wert der gebildeten Dispersion mit Schwefelsäure auf einen Bereich von 6,5 bis 7, insbesondere auf pH 6.8 abzusenken, anschließend kann dann die Überführung in ein Trockenpulver u.a. durch Sprühtrocknung, Sprühkühlung, modifizierte Sprühtrocknung, Gefriertrocknung oder Trocknung im Wirbelbett, gegebenenfalls auch in Gegenwart eines Überzugmaterials erfolgen. Als Überzugsmittel eignen sich u.a. Maisstärke, Kieselsäure, modifizierte Kieselsäure, Tricalciumphosphat (TCP), Calciumkarbonat, Natriumkarbonat, Kaliumkarbonat, Magnesiumkarbonat, Calciumoxid, Magnesiumoxid, Dicalciumdiphosphat, Calciumsilikat, Magnesiumsilikat, Magnesiumtrisilikat, Natrium-Aluminiumsilikat, Talkum, Kaolin, Calciumstearat, Magnesiumstearat, Stärken der verschiedenen botanischen Quellen, Cellulose oder Mischungen davon. Besonders bevorzugt sind dabei Siliziumdioxid, Tricalciumphosphat (TCP), hydrophob modifizierte Kieselsäure und Maisstärke.

Bevorzugt wird in Verfahrensschritt c2) die gebildete Dispersion durch destillative Abtrennung der Hauptmenge des Lösungsmittels bzw. Gemisches auf eine Feststoffkonzentration von etwa 25 bis 50 Gew-% aufkonzentriert und anschließend diese aufkonzentrierte Dispersion in einem Sprühtrockner in ein Trockenpulver überführt.

Besonders bevorzugt wird in Verfahrensschritt c2) des erfindungsgemäßen Verfahrens die Trocknung in einem Sprühtrockner mit integriertem und/oder nachgeschaltetem, externen Wirbelbett durchgeführt. Dabei wird bevorzugt eine pulverförmige Zubereitung mit agglomerierten Partikeln gebildet.

Mit Ausnahme der Abtrennung des organischen Lösungsmittels, die in Verfahrensschritt c3) nicht notwendig ist, sind die Verfahrensmaßnahmen c2) mit den genannten Bevorzugungen auch auf den Verfahrensschritt c3) anzuwenden.

Die erfindungsgemäße pulverförmige Zubereitung eignet sich u.a. als Zusatzstoff zu Lebensmittelzubereitungen, beispielsweise zur Färbung von Lebensmitteln wie Getränken, als Mittel für die Herstellung pharmazeutischer und kosmetischer Zubereitungen sowie für die Herstellung von Nahrungsergänzungspräparaten, beispielsweise von Multivitaminpräparaten im Human- und Tierbereich.

Ein weiterer Gegenstand der vorliegenden Erfindung ist die Verwendung der oben beschriebenen, erfindungsgemäßen pulverförmigen Formulierung als Zusatz zu Tierfuttermitteln, Lebensmitteln, Nahrungsergänzungsmitteln, Körperpflegeprodukten oder pharmazeutischen Mitteln.

Die Erfindung wird durch folgende, die Erfindung jedoch in keinerlei Weise einschränkende Beispiele erläutert:

### Beispiele

### Beispiel 1 (Beispiel 7 aus Tabelle 1)

30 g Citranaxanthin wurden in 240 g Isopropanol gemeinsam mit 1,1 g Ascorbylpalmitat suspendiert und bei Einstellung des Druckbegrenzungsventils auf 30 bar mit 390 g Isopropanol in einer Mischkammer A kontinuierlich gemischt. Bei einer Dosiergeschwindigkeit von 6 I/h auf der Suspensionsseite und von 9 I/h auf der Lösungsmittelseite wurde in der Mischkammer A eine Mischungstemperatur von 170°C eingestellt. Nach einer Verweilzeit von 0,3 Sekunden wurde die molekular-disperse Lösung in Mischkammer B mit einer Lösung von 32 g Gelatine, 71,4g Saccharose und 50 g Glukosesirup in 4000 g Wasser bei einer Durchsatzgeschwindigkeit von 100 I/h gemischt. Nach Abtrennen des Lösungsmittels unter vermindertem Druck in einer Destillationsapparatur wurde eine Wirkstoff-Dispersion erhalten, der 8g Sonnenblumenöl und 8g Propylgallat zugesetzt wurden. Das Propylgallat wurde hierbei in 200ml Wasser vorgelöst und mit NaOH auf pH 7 eingestellt. Anschließend wurde die Dispersion durch Sprühtrocknung in ein stabiles, wasserlösliches Trockenpulver überführt. Nach Auflösen in Wasser wurde per Photonenkorrelationsspektroskopie (PCS) eine Teilchengröße von 386 nm (Standardabweichung 142,5, Polydispersitätsindex PDI 0,185, D(95): 660nm) gemessen (Malvern Zetasizer Nano ZSP).

### Stabilitätsprüfung für Citranaxanthin

Die Stabilität der so hergestellten Partikel wurde in einem Stress-Test geprüft. Dafür wurden Muster von jeweils 100 mg der hergestellten Partikel und 4 g Prämixmischung in 50ml-Glasfläschchen eingewogen. Die Prämixmischung bestand aus 50 Gew.-% Feinkalk (Partikelgrösse <1000µm), 20 Gew.-% Weizengrießkleie (Partikelgrösse <1000µm), 20 Gew.-% 50%-igem auf Kieselsäure geträgertem Cholinchlorid (Partikelgrösse <1000µm) und 10 Gew-% Spurenelementmischung (Partikelgrösse 100-500µm) besteht, die Spurenelementmischung aus 46,78 Gew-% FeSO₄x7H₂O (100-500µm), 37,43 Gew-% CuSO₄x5H₂O (100-500µm), 11,79 Gew-% ZnO (<500µm), 3,61 Gew-% MnO und 0,39 Gew-% CoCO₃. Nach Zugabe aller Inhaltsstoffe wurden die Muster sorgfältig von Hand gemischt. Diese Muster wurden in einer Klimakammer bei 40°C und 70% Luftfeuchte für 4 Wochen gelagert. Vor Beginn der Lagerung und nach Abschluss der Lagerung wurde der Citranaxanthin-Gehalt der Muster bestimmt. Aus dem Verhältnis der Citranaxanthin Gehalte nach und vor der Lagerung wurde die Retention berechnet.

Die Retentionswerte der Beispiele sind in der folgenden Tabelle zusammengefasst:

**Tabelle 1**

| | Wirkstoff | Antioxidans | Antioxidans [Gew-%] | Zugabeort Antioxidans | Retention Wirkstoffgehalt nach 4 Wochen Test [%] |
|---|---|---|---|---|---|
| 1 | Citranaxanthin | - | 0 | Dispersion | 7,9 |
| 2 | Citranaxanthin | Ethoxyquin | 4 | als Feststoff zur Dispersion hinzugefügt, keine pH-Wert Anpassung | 52,6 |
| 3 | Citranaxanthin | BHA | 4 | Geschmolzenes BHA wurde 2 min bei 10000 U/min mit Ultraturrax in die Dispersion eingerührt und diese dann bei 1000 bar 5x über den Microfluidizer gegeben | 8,2 |
| 4 | Citranaxanthin | Na-Ascorbat | 4 | als Feststoff zur Dispersion hinzugefügt, keine pH-Wert Anpassung | 2,4 |
| 5 | Citranaxanthin | Methyl-hydrochinon | 4 | als Feststoff zur Dispersion hinzugefügt, keine pH-Wert Anpassung | 10,2 |
| 6 | Citranaxanthin | Vitamin E TPGS | 4 | Zur Dispersion hinzugefügt als 10% Lösung ohne pH-Wert Anpassung | 2,2 |
| 7 | Citranaxanthin | Propylgallat | 4 | Gelöst in Wasser pH 7 (4 Gew-% Lösung) und zur Dispersion hinzugefügt | 47,8 |
| 8 | Citranaxanthin | Lauryl gallat | 4 | Zugabe zur-Wirkstoffphase, keine pH Anpassung | 15,9 |
| 9 | Citranaxanthin | Grüner Tee Extrakt | 4 | Gelöst in Wasser pH 7 und zur Dispersion hinzugefügt | 19,4 |
| 10 | Citranaxanthin | Rosmarin säure | 4 | Zur Dispersion hinzugefügt als 10% Lösung ohne pH-Wert Anpassung | 14 |

Je höher die Retention desto besser die Stabilität der Partikel bzw. ihrer Zubereitung.

### Beispiel 2

### Einfluss des pH-Wertes auf die Propylgallat Aktivität

Die Versuche zeigen, dass ein optimaler pH-Wert Bereich für die Propylgallat Zugabe im Bereich pH 4,5 bis einschließlich pH 8,5 liegt. Selbst kurze Verweilzeiten bei pH-Werten grösser 8.5 oder ein Vorlösen des Propylgallats bei solchen pH-Werten waren ausreichend die Aktivität von Propylgallat stark einzuschränken.

**Tabelle 2a. Einfluss des pH-Werts in der Propylgallat enthaltenden Dispersion auf die Citranaxanthin Stabilität.**

| Wirkstoff | Propy-Igallat [Gew-%] | Zugabeort Propylgallat | pH Wert der versprühten Dispersion | Retention (Aktiver Bestandteil nach 4 Wochen im Test [%] |
|---|---|---|---|---|
| Citranaxanthin | 4 | Dispersion | 4,5 | 42,2 |
| Citranaxanthin | 4 | Dispersion | 6 | 39,1 |
| Citranaxanthin | 4 | Dispersion | 7 | 47,8 |
| Citranaxanthin | 4 | Dispersion | 8 | 49,4 |
| Citranaxanthin | 4 | Dispersion | 9 | 28,2 |
| Citranaxanthin | 4 | Dispersion | 10 | 36,4 |
| Citranaxanthin | 4 | Dispersion | 11 | 14,7 |

Dieses Ergebnis wird auch bestätigt, wenn das Propylgallat nicht der Vitamin enthaltenden Dispersion zugefügt wird, sondern das Propylgallat bereits der Schutzkolloidphase zugefügt wird (Tabelle 2b). Ein sehr gutes Ergebnis zeigt sich, wenn das Propylgallat der Schutzkolloidphase zugegeben wird, diese mit NaOH von einem pH Wert 5 bis 7 auf einen pH von 7 eingestellt wird und erst 15 bis 30 Minuten vor der Fällung der pH-Wert mit NaOH auf 8 bis 8.5 angehoben wird.

**Tabelle 2b. Einfluss des pH-Werts in der Propylgallat enthaltenden Schutzkolloidphase auf die Citranaxanthin Stabilität.**

| Wirkstoff | Propylgallat [Gew-%] | Zugabeort Propylgallat | pH-Wert der Kolloidphase | Retention Wirkstoffgehalt nach 4 Wochen Test [%] |
|---|---|---|---|---|
| Citranaxanthin | 4 | Kolloidphase | Voreinstellung auf 7; dann Einstellung auf pH 8.5 in Maximum | 44,1 |
| Citranaxanthin | 4 | Kolloidphase | 8.5-9.5 | 13,4 |

Die Zugabeart des Propylgallats (als Feststoff oder gelöst in Wasser pH 7) zur Vitamin enthaltenden Dispersion hat dagegen keinen Einfluß auf die Citranaxanthin Stabilität (Tabelle 2c).

**Tabelle 2c. Untersuchung der Zugabeart des Propylgallats auf die Retention**

| Wirkstoff | Propylgallat [Gew-%] | Zugabeort Propylgallat | Zugabeart des PG | Retention Wirkstoffgehalt nach 4 Wochen Test [%] |
|---|---|---|---|---|
| Citranaxanthin | 4 | Dispersion | Gelöst in Wasser pH 7 | 47,8 |
| Citranaxanthin | 4 | Dispersion | Gelöst in Wasser pH 7 | 46 |
| Citranaxanthin | 4 | Dispersion | Gelöst in Wasser pH 7 | 49,4 |
| Citranaxanthin | 4 | Dispersion | Festzugabe ohne Vorlösen | 47,2 |
| Citranaxanthin | 4 | Dispersion | Festzugabe ohne Vorlösen | 44,9 |

### Beispiel 3:

Einfluss der Propylgallat-Konzentration auf die Wirkstoffstabilität
a). Die Erhöhung der Propylgallat Konzentration erhöht die Stabilität des Wirkstoffs Citranaxanthin. Allerdings wird überraschend bei einer Erhöhung des Propylgallats in der Zubereitung auf 10 Gew-% ein starker Abfall in der Stabilität beobachtet. Ein sehr guter stabilisierender Effekt konnte bei 4 bis 9 Gew.-% Propylgallat an der Zubereitung, bevorzugt 7 bis 9 Gew.-% mit einem Optimum bei 8 bis 9 Gew.-% beobachtet werden (Tabelle 3a).

**Tabelle 3a. Einfluss der Propylgallat-Konzentration auf die Wirkstoffstabilität von Citranaxanthin**

| Wirkstoff | Propylgallat [Gew-%] | Zugabeort Propylgallat | Retention Wirkstoffgehalt nach 4 Wochen Test [%] |
|---|---|---|---|
| Citranaxanthin | 0 | Dispersion | 7,9 |
| Citranaxanthin | 2 | Dispersion | 20,2 |
| Citranaxanthin | 3 | Dispersion | 26,7 |
| Citranaxanthin | 4 | Dispersion | 47,8 |
| Citranaxanthin | 5 | Dispersion | 45,7 |
| Citranaxanthin | 6 | Dispersion | 45,9 |
| Citranaxanthin | 7 | Dispersion | 49,5 |
| Citranaxanthin | 8 | Dispersion | 55,9 |
| Citranaxanthin | 8,5 | Dispersion | 51,9 |
| Citranaxanthin | 9 | Dispersion | 57,4 |
| Citranaxanthin | 10 | Dispersion | 19,6 |

Der positive Effekt von Propylgallat, wie das Konzentrationsoptimum kann auch für andere Carotenoide bestätigt werden. (Tabellen 3b-e).

b). 30 g Canthaxanthin wurden in 240 g Isopropanol gemeinsam mit 1,1 g Ascorbylpalmitat suspendiert und bei Einstellung des Druckbegrenzungsventils auf 30 bar mit 390 g Isopropanol in einer Mischkammer A kontinuierlich gemischt. Bei einer Dosiergeschwindigkeit von 6 I/h auf der Suspensionsseite und von 9 I/h auf der Lösungsmittelseite wurde in der Mischkammer A eine Mischungstemperatur von 170°C eingestellt. Nach einer Verweilzeit von 0,3 Sekunden wurde die molekular-disperse Lösung in Mischkammer B mit einer Lösung von 32 g Gelatine und 121,4g Saccharose in 4000 g Wasser bei einer Durchsatzgeschwindigkeit von 100 I/h gemischt. Nach Abtrennen des Lösungsmittels unter vermindertem Druck in einer Destillationsapparatur wurde eine Wirkstoff-Dispersion erhalten, der 8g Sonnenblumenöl und die jeweils in Tabelle 3b angegebenen Mengen Propylgallat in Gew.-% zugesetzt wurden. Das Propylgallat wurde hierbei in 200ml Wasser vorgelöst und mit NaOH auf pH 7 eingestellt. Anschließend wurde die Dispersion durch Sprühtrocknung in ein stabiles, wasserlösliches Trockenpulver überführt. Nach Auflösen in Wasser wurde per PCS eine Teilchengröße von 290 nm (Standardabweichung 140, Polydispersitätsindex PDI 0,193, D(95): 594nm) gemessen (Malvern Zetasizer Nano ZSP).

**Tabelle 3b. Einfluss der Propylgallat-Konzentration auf die Wirkstoffstabilität von Canthaxanthin**

| Wirkstoff | Propylgallat [%] | Zugabeort Propylgallat | Retention Wirkstoffgehalt nach 4 Wochen Prämix Test [%] |
|---|---|---|---|
| Canthaxanthin | 4 | Dispersion | 61,2 |
| Canthaxanthin | 6 | Dispersion | 77,9 |
| Canthaxanthin | 8 | Dispersion | 83,3 |
| Canthaxanthin | 9 | Dispersion | 82,4 |
| Canthaxanthin | 10 | Dispersion | 69,8 |
| Canthaxanthin | 12 | Dispersion | 47,2 |

c). 30 g C30-Ester wurden in 240 g Isopropanol gemeinsam mit 1,1 g Ascorbylpalmitat suspendiert und bei Einstellung des Druckbegrenzungsventils auf 30 bar mit 390 g Isopropanol in einer Mischkammer A kontinuierlich gemischt. Bei einer Dosiergeschwindigkeit von 6 I/h auf der Suspensionsseite und von 9 I/h auf der Lösungsmittelseite wurde in der Mischkammer A eine Mischungstemperatur von 170°C eingestellt. Nach einer Verweilzeit von 0,3 Sekunden wurde die molekular-disperse Lösung in Mischkammer B mit einer Lösung von 32g Gelatine und 121,4g Saccharose in 4000 g Wasser bei einer Durchsatzgeschwindigkeit von 100 I/h gemischt. Nach Abtrennen des Lösungsmittels unter vermindertem Druck in einer Destillationsapparatur wurde eine Wirkstoff-Dispersion erhalten, der 8 g Sonnenblumenöl und die jeweils in Tabelle 3c angegebenen Mengen Propylgallat in Gew.-% zugesetzt wurden. Das Propylgallat wurde hierbei in 200ml Wasser vorgelöst und mit NaOH auf pH 7 eingestellt. Anschließend wurde die Dispersion durch Sprühtrocknung in ein stabiles, wasserlösliches Trockenpulver überführt. Nach Auflösen in Wasser wurde per PCS eine Teilchengröße von 280 nm (Standardabweichung 120, Polydispersitätsindex PDI 0,181) gemessen (Malvern Zetasizer Nano ZSP).

**Tabelle 3c. Einfluss der Propylgallat-Konzentration auf die Wirkstoffstabilität von C30-Ester**

| Wirkstoff | Propylgallat [Gew-%] | Zugabeort Propylgallat | Retention Wirkstoffgehalt nach 4 Wochen Prämix Test [%] |
|---|---|---|---|
| C30-Ester | 0 | Dispersion | 6,7 |
| C30-Ester | 2 | Dispersion | 13,83 |
| C30-Ester | 4 | Dispersion | 42,7 |
| C30-Ester | 6 | Dispersion | 57,7 |
| C30-Ester | 8 | Dispersion | 64,1 |
| C30-Ester | 10 | Dispersion | 58,5 |
| C30-Ester | 12 | Dispersion | 54,3 |

d). 30 g ß-Carotin wurden in 240 g Isopropanol gemeinsam mit 1,1 g Ascorbylpalmitat suspendiert und bei Einstellung des Druckbegrenzungsventils auf 30 bar mit 390 g Isopropanol in einer Mischkammer A kontinuierlich gemischt. Bei einer Dosiergeschwindigkeit von 6 I/h auf der Suspensionsseite und von 9 I/h auf der Lösungsmittelseite wurde in der Mischkammer A eine Mischungstemperatur von 170°C eingestellt. Nach einer Verweilzeit von 0,3 Sekunden wurde die molekular-disperse Lösung in Mischkammer B mit einer Lösung von 32 g Gelatine, 71,4g Saccharose und 50 g Glukosesirup in 4000 g Wasser bei einer Durchsatzgeschwindigkeit von 100 I/h gemischt. Nach Abtrennen des Lösungsmittels unter vermindertem Druck in einer Destillationsapparatur wurde eine Wirkstoff-Dispersion erhalten, der 8g Sonnenblumenöl und 8g Propy-Igallat zugesetzt wurden. Das Propylgallat wurde hierbei in 200ml Wasser vorgelöst und mit NaOH auf pH 7 eingestellt. Anschließend wurde die Dispersion durch Sprühtrocknung in ein stabiles, wasserlösliches Trockenpulver überführt. Nach Auflösen in Wasser wurde per PCS eine Teilchengröße von 262nm (Standardabweichung 182, Polydispersitätsindex PDI 0,268) gemessen (Malvern Zetasizer Nano ZSP).

**Tabelle 3d. Vergleich der Stabilitäten von Ethoxyquin und Propylgallat auf β-Carotin.**

| Wirkstoff | Antioxidans | Antioxidans [Gew.-%] | Zugabeort Antioxidans | Retention Wirkstoffgehalt nach 4 Wochen Test [%] |
|---|---|---|---|---|
| β-Carotin | Ethoxyquin | 4 | Dispersion | 22,2 |
| β-Carotin | Propylgallat | 4 | Dispersion | 30,3 |

e). 30 g C30-Ester wurden in 240 g Isopropanol gemeinsam mit 1,1 g Ascorbylpalmitat suspendiert und bei Einstellung des Druckbegrenzungsventils auf 30 bar mit 390 g Isopropanol in einer Mischkammer A kontinuierlich gemischt. Bei einer Dosiergeschwindigkeit von 6 I/h auf der Suspensionsseite und von 9 I/h auf der Lösungsmittelseite wurde in der Mischkammer A eine Mischungstemperatur von 170°C eingestellt. Nach einer Verweilzeit von 0,3 Sekunden wurde die molekular-disperse Lösung in Mischkammer B mit einer Lösung von 32 g Gelatine und 121,4 g Saccharose in 4000 g Wasser bei einer Durchsatzgeschwindigkeit von 100 I/h gemischt. Nach Abtrennen des Lösungsmittels unter vermindertem Druck in einer Destillationsapparatur wurde eine Wirkstoff-Dispersion erhalten, der 8g Sonnenblumenöl und und die jeweils in Tabelle 3e angegebenen Mengen Propylgallat in Gew.-% zugesetzt wurden. Das Propylgallat wurde hierbei in 200ml Wasser vorgelöst und mit NaOH auf pH 7 eingestellt. Anschließend wurde die Dispersion durch Sprühtrocknung in ein stabiles, wasserlösliches Trockenpulver überführt. Nach Auflösen in Wasser wurde per PCS eine Teilchengröße von 373 nm (Standardabweichung 165, Polydispersitätsindex PDI 0,203, D(95): 683nm) gemessen (Malvern Zetasizer Nano ZSP).

**Tabelle 3e. Vergleich der Stabilitäten von Ethoxyquin und Propylgallat auf C-30 Ester.**

| Wirkstoff | Antioxidans | Antioxidans [Gew-%] | Zugabeort Antioxidans | Retention Wirkstoffgehalt nach 4 Wochen Test [%] |
|---|---|---|---|---|
| C30 ester | Ethoxyquin | 4 | Dispersion | 28,6 |
| C30 ester | Propylgallat | 4 | Dispersion | 33,4 |
| C30 ester | Propylgallat | 4 | Dispersion (pH Wert Einstellung vor Versprühung von pH 8,5 auf pH 6.8 mit H₂SO₄ | 39,8 |
| C30 ester | Propylgallat | 6 | Dispersion | 44,4 |
| C30 ester | Propylgallat | 8 | Dispersion | 43,4 |

### Beispiel 4

Einfluss von mixed Tocopherol und D,L α-Tocopherol auf die Stabilitäten

Wegen ihrer schlechten Wasserlöslichkeit werden die aus synthetischen und aus natürlichen Quellen stammenden Tocopherole in der den Wirkstoff enthaltenden Phase gelöst. Werden jeweils jeweils nur natürliches oder synthetisches Tocopherol alleine hinzugefügt, unterscheiden sich beide in ihrer Schutzwirkung als Antioxidans und in ihrer Stabilisierung der Carotenoide bzw. Retinoide nicht.

**Tabelle 4a. Einfluss der synthetischen und natürlichen Tocopherole auf die Stabilität**

| Wirkstoff | Antioxidans | Antioxidans [Gew-%] | Zugabeort Antioxidans | Retention Wirkstoffgehalt nach 4 Wochen Test [%] |
|---|---|---|---|---|
| Canthaxanthin | Mixed Tocopherol | 4 | Wirkstoffphase | 48,7 |
| Canthaxanthin | D,L-α Tocopherol | 4 | Wirkstoffphase | 45,7 |

Anders verhält es sich, wenn zusätzlich Propylgallat als Antioxidans zu den Tocopherolen hinzugefügt wird. In diesem Falle ist die Stabilierung im Prämix-Test für die Kombination mixed Tocopherol/Propylgallat deutlich ausgeprägter als für die Kombination D,L alpha Tocopherol mit Propylgallat. (Tabelle 4b).

**Tabelle 4b. Einfluss der Kombination synthetischer oder natürlicher Tocopherole mit Propy-Igallat auf die Stabilitäten von Vitaminen.**

| Wirkstoff | Antioxidans | Antioxidans [Gew-%] | Retention Wirkstoffgehalt nach 4 Wochen Test |
|---|---|---|---|
| Canthaxanthin | Mixed Tocopherol (in Wirkstoffphase) + Propylgallat (in Schutzkolloidphase) | 4 | 78,1 |
| | | 4 | |
| Canthaxanthin | D,L-α Tocopherol (in Wirkstoffphase) + Propylgallat (in Schutzkolloidphase) | 4 | 57,2 |
| | | 4 | |
| | | | |
| C30-Ester | Mixed Tocopherol (in Wirkstoffphase) + Propylgallat (in Schutzkolloidphase) | 4 | 57,5 |
| | | 4 | |
| C30-Ester | D,L-α Tocopherol (in Wirkstoffphase) + Propylgallat (in Schutzkolloidphase) | 4 | 29,2 |
| | | 4 | |
| | | | |
| Citranaxanthin | Mixed Tocopherol (in Wirkstoffphase) + Propylgallat (in Schutzkolloidphase) | 4 | 67,5 |
| | | 4 | |
| Citranaxanthin | D,L-α Tocopherol (in Wirkstoffphase) + Propylgallat (in Schutzkolloidphase) | 4 | 21,3 |
| | | 4 | |

### Beispiel 5

### a). Synergistischer Effekt zwischen Propylgallat und Tocopherol

Für die Kombination Propylgallat mit Tocopherol wurde gefunden, dass für dieses System die Gesamtkonzentration an Antioxidans in der Zubereitung reduziert werden kann ohne an Stabilität einzubüßen. Vergleicht man die Stabilitäten für die 4% Propylgallat enthaltende Zubereitung (61,2%) mit der 4% mixed Tocopherol enthaltenden (48,7%) miteinander ist für dieses System das Propylgallat eindeutig das bessere Antioxidans. Mischt man dagegen 4% Propylgallat mit 4% mixed Tocopherol und vergleicht man dessen Stabilität (80,3%) mit der 8% Propylgallat (83,3%) enthaltenden, ist ein synergistischer Effekt erkennbar, der aus den Einzelwerten nicht zu erwarten gewesen wäre. Dies wird noch augenfälliger, wenn man bei gleichbleibendem Pro pylgallatgehalt die Konzentration an mixed Tocopherol von 4% auf 2% reduziert. Für dieses System mißt man eine Stabilität von 80,6%, während man für ein System, das 6% Propylgallat enthält nur eine Stabilität von 71,3% erhält. Dies zeigt eindrücklich, dass nur eine bestimmte Konzentration von mixed Tocopherol benötigt wird um einen starken Stabilitätsanstieg mit vermindertem Gehalt Antioxidans in der Zubereitung zu erhalten.

**Tabelle 5a. Kombination aus mixed Tocopherol mit Propylgallat.**

| Wirkstoff | Propylgallat [Gew.-%] | mixed Tocopherol [Gew.-%] | Retention Canthaxanthin nach 4 Wochen Test [%] |
|---|---|---|---|
| Canthaxanthin | 4 | 0 | 61,2 |
| Canthaxanthin | 6 | 0 | 71,3 |
| Canthaxanthin | 8 | 0 | 83,3 |
| Canthaxanthin | 0 | 4 | 48,7 |
| Canthaxanthin | 4 | 4 | 80,3 |
| Canthaxanthin | 4 | 2 | 80,8 |

2 Gew-% mixed Tocopherol in Kombination mit 4 Gew-% Propylgallat erreichen fast den Stabilitätswert von 4 Gew-% mixed Tocopherol in Kombination mit 4 Gew-% Propylgallat. Reduziert man den Anteil von mixed Tocopherol jedoch auf 1 Gew-%, wiederum in Kombination mit 4 Gew-% Propylgallat beginnen die Stabilitätswerte zu sinken. (Tabelle 5b).

**Tabelle 5b. Kombination von mixed Tocopherol mit Propylgallat.**

| Wirkstoff | Propylgallat [Gew-%] | mixed Tocopherol [Gew-%] | Retention Canthaxanthin nach 4 Wochen Test [%] |
|---|---|---|---|
| Canthaxanthin | 4 (in Schutzkolloidphase pH7) | 2 (in Wirkstoffphase) | 76 |
| Canthaxanthin | 4 (in Schutzkolloidphase pH7) | 4 (in Wirkstoffphase) | 80,6 |
| Canthaxanthin | 4 (in Schutzkolloidphase pH7) | 1 (in Wirkstoffphase) | 72,8 |

**Tabelle 5c. Kombination von mixed Tocopherol mit Propylgallat**

| Wirkstoff | Propylgallat [Gew-%] | mixed Tocopherol [Gew-%] | Retention Canthaxanthin nach 4 Wochen Test [%] |
|---|---|---|---|
| C30 Ester | 4 | 0 | 42,7 |
| C30 Ester | 0 | 4 | 11 |
| C30 Ester | 4 | 4 | 61,7 |
| | | | |
| Citranaxanthin | 4 | 0 | 39,1 |
| Citranaxanthin | 0 | 4 | 10,8 |
| Citranaxanthin | 4 | 4 | 66,5 |

### Versuchsvorschrift für die Zugabe von 4 Gew.-% Propylgallat und 4 Gew.-% Tocopherol

30 g Citranaxanthin wurden in 240 g Isopropanol gemeinsam mit 1,1 g Ascorbylpalmitat und 9 g Mixed Tocopherol suspendiert und bei Einstellung des Druckbegrenzungsventils auf 30 bar mit 390 g Isopropanol in einer Mischkammer A kontinuierlich gemischt. Bei einer Dosiergeschwindigkeit von 6 I/h auf der Suspensionsseite und von 9 I/h auf der Lösungsmittelseite wurde in der Mischkammer A eine Mischungstemperatur von 170°C eingestellt. Nach einer Verweilzeit von 0,3 Sekunden wurde die molekular-disperse Lösung in Mischkammer B mit einer auf pH 9 eingestellten Lösung von 32 g Gelatine, 71,4 g Saccharose, 50 g Glukosesirup und 9g Propylgallat in 4100 g Wasser bei einer Durchsatzgeschwindigkeit von 100 I/h gemischt. Nach Abtrennen des Lösungsmittels unter vermindertem Druck in einer Destillationsapparatur wurde eine Wirkstoff-Dispersion erhalten, der 9g Sonnenblumenöl zugesetzt wurden. Anschließend wurde die Dispersion durch Sprühtrocknung in ein stabiles, wasserlösliches Trockenpulver überführt. Nach Auflösen in Wasser wurde per PCS eine Teilchengröße von 280 nm (Standardabweichung 142,5, Polydispersitätsindex PDI 0,185) gemessen (Malvern Zetasizer Nano ZSP).

### b). Synergistischer Effekt zwischen Propylgallat und BHT

**Tabelle 5d. Kombination aus BHT mit Propylgallat.**

| Wirkstoff | Propylgallat [Gew-%] | BHT [Gew.-%] | Retention Citranaxanthin nach 4 Wochen Test [%] |
|---|---|---|---|
| Citranaxanthin | 4 | 0 | 61,2 |
| Citranaxanthin | 4 | 4 | 82,3 |
| Citranaxanthin | 0 | 4 | 56,1 |
| Citranaxanthin | 0 | 4 | 65,5 |
| Citranaxanthin | 0 | 4 | 67,9 |
| Citranaxanthin | 4 Gew.-% Ethoxyquin | | 79,3 |

### Versuchsvorschrift für die Zugabe von 4 Gew.-% Propylgallat und 4 Gew.-% Butylhydroxytoluol

30 g Citranaxanthin werden in 240 g Isopropanol gemeinsam mit 1,1 g Ascorbylpalmitat suspendiert und bei Einstellung des Druckbegrenzungsventils auf 30 bar mit 390 g Isopropanol in einer Mischkammer A kontinuierlich gemischt. Bei einer Dosiergeschwindigkeit von 6 I/h auf der Suspensionsseite und von 9 I/h auf der Lösungsmittelseite wird in der Mischkammer A eine Mischungstemperatur von 170°C eingestellt. Nach einer Verweilzeit von 0,3 Sekunden wird die molekular-disperse Lösung in Mischkammer B mit einer auf pH 9 eingestellten Lösung von 32 g Gelatine, 71,4 g Saccharose, 50 g Glukosesirup und 9 g Propylgallat in 4100 g Wasser bei einer Durchsatzgeschwindigkeit von 100 I/h gemischt. Nach Abtrennen des Lösungsmittels unter vermindertem Druck in einer Destillationsapparatur wird eine Wirkstoff-Dispersion erhalten, der 9g Sonnenblumenöl und darin gelöste 9g BHT zugesetzt werden.

Anschließend wurde die Dispersion durch Sprühtrocknung in ein stabiles, wasserlösliches Trockenpulver überführt. Nach Auflösen in Wasser wurde per PCS eine Teilchengröße von 290 nm (Standardabweichung 140, Polydispersitätsindex PDI 0,180) gemessen (Malvern Zetasizer Nano ZSP).

## Patentansprüche

1. Pulverförmige Vitaminzubereitung, in denen das Vitamin im Wesentlichen eine Teilchengrösse von weniger als 0,7 µm besitzt, **dadurch gekennzeichnet dass** als wirksame Menge 3,5 bis 9,5 Gew.-% Propylgallat bezogen auf die Gesamtmenge der Zubereitung eingesetzt werden, wobei das Gewichtsverhältnis von Propylgallat zu Vitamin bei der Herstellung zwischen 0,21 und 2,63 liegt.

2. Pulverförmige Vitaminzubereitung nach Anspruch 1, **dadurch gekennzeichnet, dass** das Vitamin ausgewählt ist aus der Gruppe bestehend aus Vitamine A, D, E, K oder Q oder deren Derivate, beispielsweise Vitamin A- und Vitamin E- Ester wie Retinylacetat oder Tocopherolacetat, Tocotrienol, Vitamin K1, Vitamin K2, Coenzym Q10 sowie Carotinoide, wie ß-Carotin, Canthaxanthin, Citranaxanthin, Astaxanthin und Esterderivate, Zeaxanthin und Esterderivate, Lutein und Esterderivate, Lycopin, Apocarotinsäure und Esterderivate, Apocarotinal und Mischungen davon.

3. Pulverförmige Vitaminzubereitung nach den Ansprüchen 1 oder 2, **dadurch gekennzeichnet, dass** die Zubereitung Butylhydroxytoluol oder synthetisches und/oder natürliches Tocopherol enthält.

4. Pulverförmige Vitaminzubereitung nach Anspruch 3, **dadurch gekennzeichnet, dass** das Tocopherol natürliches Tocopherol ist.

5. Pulverförmige Vitaminzubereitung nach Anspruch 3 oder 4, **dadurch gekennzeichnet, dass** Propylgallat und Tocopherol in der Formulierung in einem Verhältnis von 9:1 zu 1:2 vorliegen.

6. Pulverförmige Vitaminzubereitung nach Anspruch 3, **dadurch gekennzeichnet, dass** Propylgallat und Butylhydroxytoluol in der Formulierung in einem Verhältnis von 9:1 zu 1:2 vorliegen.

7. Pulverförmige Vitaminzubereitung nach einem der vorangehenden Ansprüche 1 bis 6, **dadurch gekennzeichnet, dass** ihr Quotient aus aktivem Vitamin (A2/A1) im Verhältnis zu einer Vergleichsprobe, die anstatt Propylgallat, die gleiche Menge Ethoxyquin enthält (B2/B1), nach 4 Wochen im Stress-Test mindestens 0,75 beträgt, wobei der Anteil an aktivem Vitamin ermittelt wird, indem man jeweils 100 mg der hergestellten Formulierung und 4 g einer Mischung aus 50 Gew.-% Feinkalk (<1000µm), 20 Gew-% Weizengrießkleie (<1000µm), 20 Gew-% 50%-igem auf Kieselsäure geträgertem Cholinchlorid (<1000µm) und 10 Gew-% Spurenelementmischung (100-500µm), die Spurenelementmischung besteht aus 46,78 Gew-% FeSO₄x7H₂O (100-500µm), 37,43 Gew-% CuSO₄x5H₂O (100-500µm), 11,79 Gew-% ZnO (<500µm), 3,61 Gew-% MnO und 0,39 Gew-% CoCO₃ in 50ml-Glasbehälter einwiegt, die Inhaltsstoffe vermischt und in einer Klimakammer bei 40° C und 70% Luftfeuchte für 4 Wochen lagert, wobei vor Beginn der Lagerung der Vitamingehalt (A1) und (B1) und nach Abschluss der Lagerung der Vitamingehalt (A2) und (B2) bestimmt wird und sich der Anteil an aktivem Vitamin aus dem Quotienten A2/A1 und B2/B1 ergibt.

8. Verfahren zur Herstellung von feinverteilten, pulverförmigen Vitaminzubereitungen, in denen das Vitamin im Wesentlichen eine Teilchengrösse von weniger als 0,7 µm besitzt, umfassend die Schritte
a1) Lösen der Vitamine in einem flüchtigen mit Wasser mischbaren organischen Lösungsmittel oder in einer Mischung aus Wasser und einem mit Wasser mischbaren organischen Lösungsmittel bei Temperaturen zwischen 50° C und 200° C, gegebenenfalls bei erhöhtem Druck, innerhalb einer Zeit von weniger als 10 Sekunden
b1) schnelles Mischen der nach a) erhaltenen Lösung mit einer wässrigen oder kolloiddispersen Lösung eines Kolloids bei Temperaturen zwischen 0° C und 50° C, wobei das Vitamin in kolloiddisperser Form ausgefällt wird,
c1) Überführung der gebildeten Dispersion in ein Trockenpulver durch Abtrennung der Hauptmenge an Lösungsmittel und anschließender Trocknung
oder
a2) Lösen der Vitamine in einem flüchtigen mit Wasser nicht mischbaren organischen Lösungsmittel bei Temperaturen von 30 bis 150° C, gegebenenfalls bei erhöhtem Druck,
b2) Mischen der nach a) erhaltenen Lösung mit einer wässrigen oder kolloiddispersen Lösung eines Kolloids, wobei sich eine Emulsion ausbildet,
c2) Entfernung des organischen Lösungsmittels aus der Emulsion und Überführung der gebildeten Suspension/Dispersion durch Abtrennung des Wassers und anschließender Trocknung in ein Trockenpulver,
oder
a3) Überführen des Vitamins, in eine Flüssigkeit durch Erwärmen über seinen Schmelzpunkt oder Lösen in einem Öl oder inkubieren bei Raumtemperatur,
b3) Mischen der nach a3) erhaltenen Schmelze mit einer wässrigen oder kolloiddispersen Lösung eines Kolloids, wobei sich eine Emulsion ausbildet,
c3) Überführung der gebildeten Dispersion durch Abtrennung des Wassers und anschließender Trocknung in ein Trockenpulver,
**dadurch gekennzeichnet, dass** das Verfahren in Gegenwart einer wirksamen Menge Propylgallat durchgeführt wird, wobei als wirksame Menge 3,5 bis 9,5 Gew.-% Propylgallat bezogen auf die Gesamtmenge der Zubereitung eingesetzt werden und das Gewichtsverhältnis von Propylgallat zu Vitamin bei der Herstellung zwischen 0,21 und 2,63 liegt.

9. Verfahren nach den Anspruch 8, **dadurch gekennzeichnet dass** das Propylgallat der organisches Lösungsmittel enthaltenden Vitamin Phase und/oder der Kolloid enthaltenden wässrigen Lösung und/oder der sich ausgebildeten Dispersion zugesetzt wird.

10. Verfahren nach den Ansprüchen 8 oder 9, **dadurch gekennzeichnet, dass** das Verfahren bei einem pH-Wert von pH 4,5 bis pH 8,5, besonders bevorzugt bei einem pH-Wert von 6,5 bis 8,5 durchgeführt wird.

11. Verfahren nach Anspruch 10, **dadurch gekennzeichnet, dass** nach Zugabe des Propylgallats zur Kolloid enthaltenden wässrigen Lösung, diese auf einen pH-Wert von 6,5 bis 8.5 eingestellt wird.

12. Verfahren nach einem der Ansprüche 8 bis 11, **dadurch gekennzeichnet, dass** der organisches Vitamin enthaltenden Phase Tocopherol zugesetzt wird.

13. Verfahren nach Anspruch 12, **dadurch gekennzeichnet, dass** das Tocopherol natürliches Tocopherol ist.

14. Verfahren nach Anspruch 12 oder 13, **dadurch gekennzeichnet, dass** Propylgallat und Tocopherol in einem Verhältnis von 9:1 zu 1:2 eingesetzt werden.

15. Verfahren nach einem der Ansprüche 8 bis 11, **dadurch gekennzeichnet, dass** der gebildeten Dispersion Butylhydroxytoluol zugesetzt wird.

16. Verfahren nach Anspruch 15, **dadurch gekennzeichnet, dass** Propylgallat und Butylhydroxytoluol in einem Verhältnis von 9:1 zu 1:2 eingesetzt werden.

17. Verfahren nach einem der Ansprüche 8 bis 16, **dadurch gekennzeichnet, dass** das Vitamin ausgewählt ist aus der Gruppe bestehend aus Vitamine A, D, E, K oder Q oder deren Derivate, beispielsweise Vitamin A- und Vitamin E- Ester wie Retinylacetat oder Tocopherolacetat, Tocotrienol, Vitamin K1, Vitamin K2, Coenzym Q10 sowie Carotinoide, wie β-Carotin, Canthaxanthin, Citranaxanthin, Astaxanthin und Esterderivate, Zeaxanthin und Esterderivate, Lutein und Esterderivate, Lycopin, Apocarotinsäure und Esterderivate und Apocarotinal.

18. Verfahren nach einem der Ansprüche 8 bis 17, **dadurch gekennzeichnet, dass** das Kolloid ausgewählt ist aus der Gruppe bestehend aus Pflanzengummis, modifizierten Pflanzengummis, Gelatine, modifizierter Gelatine, modifizierter Stärke, Ligninsulfonat, Chitosan, Carrageenan, Casein, Caseinat, Molkeprotein, Zein, modifizierter Cellulose, Pectin, modifiziertem Pectin, Pflanzenproteinen und modifizierten Pflanzenproteinen oder Mischungen davon.

19. Pulverförmige Vitaminzubereitung hergestellt nach einem Verfahren gemäß einem der Ansprüche 8 bis 18.

20. Verwendung der pulverförmigen Vitaminzubereitung gemäß einem der Ansprüche 1 bis 7 und 19 als Zusatz zu Tierfuttermitteln, Lebensmitteln, Nahrungsergänzungsmitteln, Körperpflegeprodukten oder pharmazeutischen Mitteln.

## Claims

1. A pulverulent vitamin formulation in which the vitamin essentially has a particle size of less than 0.7 µm, wherein the effective amount used is 3.5% to 9.5% by weight of propyl gallate based on the total amount of the formulation, where the weight ratio of propyl gallate to vitamin in the production is between 0.21 and 2.63.

2. The pulverulent vitamin formulation according to claim 1, wherein the vitamin is selected from the group consisting of vitamins A, D, E, K or Q or derivatives thereof, for example vitamin A esters and vitamin E esters such as retinyl acetate or tocopherol acetate, tocotrienol, vitamin K1, vitamin K2, coenzyme Q10 and carotenoids such as β-carotene, canthaxanthin, citranaxanthin, astaxanthin and ester derivatives, zeaxanthin and ester derivatives, lutein and ester derivatives, lycopene, apocarotenic acid and ester derivatives, apocarotenal and mixtures thereof.

3. The pulverulent vitamin formulation according to claim 1 or 2, wherein the formulation comprises butylhydroxytoluene or synthetic and/or natural tocopherol.

4. The pulverulent vitamin formulation according to claim 3, wherein the tocopherol is natural tocopherol.

5. The pulverulent vitamin formulation according to claim 3 or 4, wherein propyl gallate and tocopherol are present in the formulation in a ratio of 9:1 to 1:2.

6. The pulverulent vitamin formulation according to claim 3, wherein propyl gallate and butylhydroxytoluene are present in the formulation in a ratio of 9:1 to 1:2.

7. The pulverulent vitamin formulation according to any of the preceding claims 1 to 6, wherein the quotient therein of active vitamin (A2/A1) in relation to a comparative sample that comprises, rather than propyl gallate, the same amount of ethoxyquin (B2/B1), after 4 weeks in a stress test, is at least 0.75, where the proportion of active vitamin is ascertained by weighing 100 mg in each case of the formulation produced and 4 g of a mixture of 50% by weight of fine lime (< 1000 µm), 20% by weight of wheat bran (< 1000 µm), 20% by weight of 50% silica-supported choline chloride (< 1000 µm) and 10% by weight of trace element mixture (100-500 µm), said trace element mixture consisting of 46.78% by weight of FeSO₄x7H₂O (100-500 µm), 37.43% by weight of CuSO₄x5H₂O (100-500 µm), 11.79% by weight of ZnO (< 500 µm), 3.61% by weight of MnO and 0.39% by weight of CoCO₃ into 50 mL glass containers, mixing the ingredients and storing them in a climate-controlled chamber at 40°C and 70% humidity for 4 weeks, with determination of the vitamin content (A1) and (B1) prior to commencement of the storage and of the vitamin content (A2) and (B2) on conclusion of the storage, calculating the proportion of active vitamin from the quotient A2/A1 and B2/B1.

8. A process for producing finely divided, pulverulent vitamin formulations in which the vitamin essentially has a particle size of less than 0.7 µm, comprising the steps of
a1) dissolving the vitamins in a volatile, water-miscible organic solvent or in a mixture of water and a water-miscible organic solvent at temperatures between 50°C and 200°C, optionally under elevated pressure, within a period of less than 10 seconds,
b1) rapidly mixing the solution obtained after a) with an aqueous or colloidally dispersed solution of a colloid at temperatures between 0°C and 50°C, with precipitation of the vitamin in colloidally dispersed form,
c1) converting the dispersion formed to a dry powder by removing the majority of solvent and then drying,
or
a2) dissolving the vitamins in a volatile, water-immiscible organic solvent at temperatures of 30 to 150°C, optionally under elevated pressure,
b2) mixing the solution obtained after a) with an aqueous or colloidally dispersed solution of a colloid, forming an emulsion,
c2) removing the organic solvent from the emulsion and converting the suspension/dispersion formed to a dry powder by removing the water and then drying,
or
a3) converting the vitamin to a liquid by heating above its melting point or dissolving it in an oil or incubating it at room temperature,
b3) mixing the melt obtained after a3) with an aqueous or colloidally dispersed solution of a colloid, forming an emulsion,
c3) converting the dispersion formed to a dry powder by removing the water and then drying,
wherein the process is conducted in the presence of an effective amount of propyl gallate, where the effective amount used is 3.5% to 9.5% by weight of propyl gallate based on the total amount of the formulation and the weight ratio of propyl gallate to vitamin in the production is between 0.21 and 2.63.

9. The process according to claim 8, wherein the propyl gallate is added to the organic solvent-containing vitamin phase and/or to the colloid-containing aqueous solution and/or to the dispersion formed.

10. The process according to claim 8 or 9, wherein the process is conducted at a pH of pH 4.5 to pH 8.5, more preferably at a pH of 6.5 to 8.5.

11. The process according to claim 10, wherein, after addition of the propyl gallate to the colloid-containing aqueous solution, said solution is adjusted to a pH of 6.5 to 8.5.

12. The process according to any of claims 8 to 11, wherein tocopherol is added to the organic vitamin-containing phase.

13. The process according to claim 12, wherein the tocopherol is natural tocopherol.

14. The process according to claim 12 or 13, wherein propyl gallate and tocopherol are used in a ratio of 9:1 to 1:2.

15. The process according to any of claims 8 to 11, wherein butylhydroxytoluene is added to the dispersion formed.

16. The process according to claim 15, wherein propyl gallate and butylhydroxytoluene are used in a ratio of 9:1 to 1:2.

17. The process according to any of claims 8 to 16, wherein the vitamin is selected from the group consisting of vitamins A, D, E, K or Q or derivatives thereof, for example vitamin A esters and vitamin E esters such as retinyl acetate or tocopherol acetate, tocotrienol, vitamin K1, vitamin K2, coenzyme Q10 and carotenoids such as β-carotene, canthaxanthin, citranaxanthin, astaxanthin and ester derivatives, zeaxanthin and ester derivatives, lutein and ester derivatives, lycopene, apocarotenic acid and ester derivatives and apocarotenal.

18. The process according to any of claims 8 to 17, wherein the colloid is selected from the group consisting of plant gums, modified plant gums, gelatin, modified gelatin, modified starch, lignosulfonate, chitosan, carrageenan, casein, caseinate, whey protein, zein, modified cellulose, pectin, modified pectin, plant proteins and modified plant proteins or mixtures thereof.

19. A pulverulent vitamin formulation produced by a process according to any of claims 8 to 18.

20. The use of the pulverulent vitamin formulation according to any of claims 1 to 7 and 19 as additives in animal feeds, foods, food supplements, personal care products or pharmaceutical compositions.

## Revendications

1. Préparation vitaminique en poudre dans laquelle la vitamine a essentiellement une taille de particules inférieure à 0,7 µm, **caractérisée en ce que** l'on utilise comme quantité efficace 3,5 à 9,5 % en poids de gallate de propyle par rapport à la quantité totale de la préparation, le rapport pondéral gallate de propyle à vitamine dans la fabrication étant compris entre 0,21 et 2,63.

2. Préparation vitaminique en poudre selon la revendication 1, **caractérisée en ce que** la vitamine est choisie dans le groupe constitué par les vitamines A, D, E, K ou Q ou leurs dérivés, par exemple les esters de vitamine A et de vitamine E tels que l'acétate de rétinyle ou l'acétate de tocophérol, le tocotriénol, la vitamine K1, la vitamine K2, la coenzyme Q10 et les caroténoïdes, tels que le β-carotène, la canthaxanthine, la citranaxanthine, l'astaxanthine et les dérivés esters, la zéaxanthine et les dérivés esters, la lutéine et les dérivés esters, le lycopène, l'acide apocaroténique et les dérivés esters, l'apocaroténal et leurs mélanges.

3. Préparation vitaminique en poudre selon la revendication 1 ou 2, **caractérisée en ce que** la préparation contient du butylhydroxytoluène ou du tocophérol synthétique et/ou naturel.

4. Préparation vitaminique en poudre selon la revendication 3, **caractérisée en ce que** le tocophérol est le tocophérol naturel.

5. Préparation vitaminique en poudre selon la revendication 3 ou 4, **caractérisée en ce que** le gallate de propyle et le tocophérol sont présents dans la formulation dans un rapport de 9:1 à 1:2.

6. Préparation vitaminique en poudre selon la revendication 3, **caractérisée en ce que** le gallate de propyle et le butylhydroxytoluène sont présents dans la formulation dans un rapport de 9:1 à 1:2.

7. Préparation vitaminique en poudre selon l'une des revendications 1 à 6 précédentes, **caractérisée en ce que** son quotient de vitamine active (A2/A1) par rapport à un échantillon de comparaison contenant la même quantité d'éthoxyquine (B2/B1) au lieu de gallate de propyle, après 4 semaines d'épreuve de stress est d'au moins 0,75, la proportion de vitamine active étant déterminée en pesant 100 mg de la formulation préparée et 4 g d'un mélange de 50 % en poids de chaux fine (< 1 000 µm), 20 % en poids de son de semoule de blé (< 1 000 µm), 20 % en poids de chlorure de choline à 50 % supporté sur silice (< 1 000 µm) et 10 % en poids d'un mélange d'oligo-éléments (100-500 µm), le mélange d'oligo-éléments étant constitué de 46,78 % en poids de FeSO₄x7H₂O (100-500 µm), 37,43 % en poids de CuSO₄x5H₂O (100-500 µm), 11,79 % en poids de ZnO (< 500 µm), 3,61 % en poids de MnO et 0,39 % en poids de CoCO₃, dans un récipient en verre de 50 ml, en mélangeant les ingrédients et en les stockant dans une enceinte climatique à 40 °C et 70 % d'humidité de l'air pendant 4 semaines, la teneur en vitamine (A1) et (B1) étant déterminée avant le début du stockage et la teneur en vitamine (A2) et (B2) étant déterminée après la fin du stockage, et la proportion de vitamine active étant déterminée à partir des quotients A2/A1 et B2/B1.

8. Procédé de fabrication de préparations vitaminiques en poudre finement distribuées, dans lequel la vitamine a essentiellement une taille de particules inférieure à 0,7 µm, comprenant les étapes
a1) solubilisation des vitamines dans un solvant organique volatil miscible à l'eau ou dans un mélange d'eau et d'un solvant organique miscible à l'eau à des températures comprises entre 50 °C et 200 °C, le cas échéant à pression élevée, dans un délai inférieur à 10 secondes
b1) mélange rapide de la solution obtenue conformément à (a) avec une solution dispersée aqueuse ou dispersée colloïdale d'un colloïde à des températures comprises entre 0 °C et 50 °C, la vitamine étant précipitée sous forme dispersée colloïdale ;
c1) transfert de la dispersion formée dans une poudre sèche par séparation de la quantité principale de solvant, puis séchage
ou
a2) dissolution des vitamines dans un solvant organique volatil non miscible à l'eau à des températures de 30 à 150 °C, le cas échéant à pression élevée,
b2) mélange de la solution obtenue conformément à (a) avec une solution aqueuse ou dispersée colloïdale d'un colloïde, une émulsion se formant,
c2) élimination du solvant organique de l'émulsion et transfert de la suspension/dispersion formée par séparation de l'eau puis séchage en une poudre sèche, ou
a3) transfert de la vitamine dans un liquide par chauffage au-dessus de sa température de fusion ou dissolution dans une huile ou incubation à température ambiante,
b3) mélange de la masse fondue obtenue conformément à a3) avec une solution aqueuse ou dispersée colloïdale d'un colloïde, une émulsion se formant,
c3) transfert de la dispersion formée par séparation de l'eau puis séchage en une poudre sèche,
**caractérisé en ce que** le procédé est réalisé en présence d'une quantité efficace de gallate de propyle, 3,5 à 9,5 % en poids de gallate de propyle étant utilisé en tant que quantité efficace par rapport à la quantité totale de la préparation et le rapport pondéral du gallate de propyle à la vitamine dans la fabrication étant compris entre 0,21 et 2,63.

9. Procédé selon la revendication 8, **caractérisé en ce que** le gallate de propyle est ajouté à la phase vitaminique contenant un solvant organique et/ou à la solution aqueuse contenant un colloïde et/ou à la dispersion formée.

10. Procédé selon la revendication 8 ou 9, **caractérisé en ce que** le procédé est mis en œuvre à une valeur de pH de 4,5 à 8,5, de manière particulièrement préférée à une valeur de pH de 6,5 à 8,5.

11. Procédé selon la revendication 10, **caractérisé en ce qu'**après l'ajout du gallate de propyle à la solution aqueuse contenant un colloïde, celle-ci est ajustée à une valeur de pH de 6,5 à 8,5.

12. Procédé selon l'une des revendications 8 à 11, **caractérisé en ce que** du tocophérol est ajouté à la phase organique contenant des vitamines.

13. Procédé selon la revendication 12, **caractérisé en ce que** le tocophérol est le tocophérol naturel.

14. Procédé selon la revendication 12 ou 13, **caractérisé en ce que** l'on utilise du gallate de propyle et du tocophérol dans un rapport de 9:1 à 1:2.

15. Procédé selon l'une des revendications 8 à 11, **caractérisé en ce que** du butylhydroxytoluène est ajouté à la dispersion formée.

16. Procédé selon la revendication 15, **caractérisé en ce que** l'on utilise du gallate de propyle et du butylhydroxytoluène dans un rapport de 9:1 à 1:2.

17. Procédé selon l'une des revendications 8 à 16, **caractérisé en ce que** la vitamine est choisie dans le groupe constitué par les vitamines A, D, E, K ou Q ou leurs dérivés, par exemple les esters de vitamine A et de vitamine E tels que l'acétate de rétinyle ou l'acétate de tocophérol, le tocotriénol, la vitamine K1, la vitamine K2, la coenzyme Q10 et les caroténoïdes, tels que le β-carotène, la canthaxanthine, la citranaxanthine, l'astaxanthine et les dérivés esters, la zéaxanthine et les dérivés ester, la lutéine et les dérivés ester, le lycopène, l'acide apocaroténique et les dérivés d'ester et l'apocaroténal.

18. Procédé selon l'une des revendications 8 à 17, **caractérisé en ce que** le colloïde est choisi dans le groupe constitué par le caoutchouc végétal, le caoutchouc végétal modifié, la gélatine, la gélatine modifiée, l'amidon modifié, le sulfonate de lignine, le chitosan, la carraghénane, la caséine, le caséinate, la protéine de lactosérum, la zéine, la cellulose modifiée, la pectine, la pectine modifiée, les protéines végétales et les protéines végétales modifiées ou leurs mélanges.

19. Préparation vitaminique en poudre fabriquée par un procédé selon l'une des revendications 8 à 18.

20. Utilisation de la préparation vitaminique en poudre selon l'une des revendications 1 à 7 et 19 comme additif pour aliments pour animaux, aliments, compléments alimentaires, produits de soins corporels ou agents pharmaceutiques.
